# EUROPEAN PATENT APPLICATION

(11) **EP 3 141 221 A1**
(43) Date of publication of application: **15.03.2017**
(21) Application number: 16175197.9
(22) Date of filing: 20.06.2016
(51) Int. Cl.: A61F 2/30, A61F 2/42

(54) **IMPLANT AND METHOD FOR MAKING SUCH AN IMPLANT**

(30) Priority: 18.08.2015 US 201514828817
(71) Applicant: Wright Medical Technology, Inc., Memphis, TN 38117 (US)
(72) Inventor: GUILFORD, Jennifer, Memphis, TN 38104 (US); WOODARD, Joseph Ryan, Memphis, TN 38120 (US); ROBINSON, Chris, Hernando, MS 38632 (US); MOSELEY, Jon Pope, ARLINGTON, TN 38002 (US); COHEN, MD, Bruce, Charlotte, NC 28209 (US); ANDERSON, MD, Robert, CHARLOTTE, NC 28211 (US); HYER, DPM, Chris, Westerville, OH 43082 (US); LEE, MD, Thomas, Pickerington, OH 43147 (US)
(74) Representative: Lavoix

(57) **Abstract**

The implant includes a body having a cylindrical portion extending from a first end to a second end, a first nub extending from the first end, and a second nub extending from the second end. The body includes an inner layer, a middle layer, and an outer layer. The inner layer has a first length extending from a tip of the first nub to a tip of the second nub, and has a first central axis collinear with a central longitudinal axis of the implant. The outer layer has a second length extending from the first end to the second end, has a second central axis collinear with the central longitudinal axis, and provides an outer surface of the implant. The middle layer comprises a porous material disposed between the inner layer and the outer layer. The inner layer and at least one portion of the middle layer provide the two nubs.

## Description

The present invention relates to an implant and a method for making such an implant. The present disclosure relates generally to orthopedic medical implant devices for surgical joint fusion. More particularly, the disclosed subject matter relates to a joint fusion implant for the bones of the human foot, especially the metatarsophalangeal joints.

### BACKGROUND

Orthopedic implant devices have been utilized to fully or partially replace existing skeletal joints in humans. There are many joints in the human foot, and the metatarsophalangeal (MTP) joint is one joint causing frequent problems.

The MTP joint is the joint between the head of a metatarsal bone and the base of a proximal phalange in a foot. A number of efforts have been made to partially or fully replace this joint. A metatarsophalangeal (MTP) implant is used for replacing a metatarsophalangeal joint. The efforts include partial or full replacement of the joint using silicone based materials or metal implant devices.

### SUMMARY OF INVENTION

The present disclosure provides an orthopedic implant device for surgical joint fusion. More particularly, the present disclosure provides an implant such as a metatarsophalangeal (MTP) implant, and a method for making the implant. The present disclosure also provides a method of using the implant including surgical procedure for implanting the implant, for example, in a foot of a patient. These include, but are not limited to, the following aspects and embodiments.

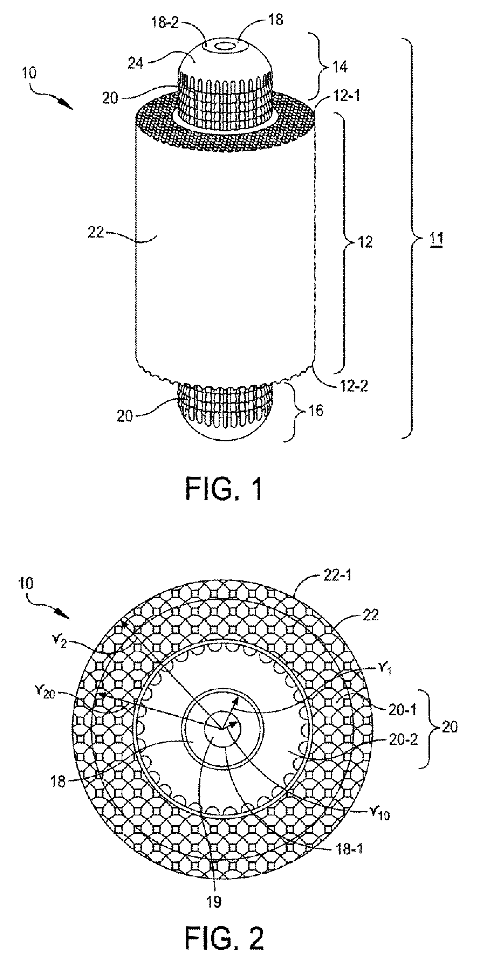

In one aspect, an orthopedic implant device (or an implant) is provided. An exemplary implant is described in details in FIGS. 1-3. The implant comprises a body having a cylindrical portion extending from a first end to a second end, a first nub extending from the first end of the cylindrical portion, and a second cylindrical portion of smaller diameter extending from the second end of the cylindrical portion. The body includes three concentric portions or layers. The inner layer has a first length extending from a tip of the first nub to a tip of the second nub, and has a first central axis that is collinear with a central longitudinal axis of the implant. The outer layer has a second length extending from the first end to the second end of the cylindrical portion, and has a second central axis that is collinear with the central longitudinal axis of the implant. The outer layer provides an outer surface of the implant. The middle layer comprises a porous material, and is disposed between the inner layer and the outer layer. The inner layer and at least one portion of the middle layer provide the two nubs.

In some embodiments, the inner layer and the outer layer have a solid or substantially solid structure, or closed nonporous structure to prevent soft tissue ingrowth. Each of the inner layer and the outer layer comprises a metal, for example, titanium, titanium alloy, or stainless steel. In some embodiments, such a metal is titanium, or titanium alloy.

In some embodiments, the middle layer comprises two portions: the at least one portion of the middle layer providing the two nubs, and a portion ("middle portion" or "sandwiched portion"), which extends from the first end to the second end of the cylindrical portion, and is sandwiched between the inner layer and the outer layer. At least one portion of the middle layer providing the two nubs is porous. The "middle portion" (or "sandwiched portion") can be porous or solid. The two portions of the middle layer are one unitary layer comprising a porous material in some embodiments. The middle layer may comprise porous titanium or titanium alloy. The porous material in the middle layer may have pores of any suitable size. The pore size may be in the range of from about 1 micron to about 2000 microns in diameter. In some embodiments, the pore size is higher than 5 microns, for example, from about 5 microns to about 100 microns in diameter, or from about 50 microns to about 1000 microns in diameter, or from about 400 microns to about 600 microns in diameter.

In some embodiments, the middle layer has at least one exposed surface having a predetermined surface roughness and configured to promote bone fixation through friction and bone ingrowth. In some embodiments, the middle layer may have a region with a smooth surface adjacent to the tip of the nubs or the end surfaces of the inner layer.

Examples of the implant provided in the present disclosure include but are not limited to a metatarsophalangeal (MTP) implant configured to fuse, fix or partially replace a metatarsophalangeal joint of a patient. Such an implant may be any other suitable implant configured to fuse, fix or partially replace a joint between two bones.

In another aspect, a method for making the implant described above is also provided. In some embodiments, the method comprises the following steps: forming an article for the implant using the technique of additive manufacturing, and optionally sintering the article at an elevated temperature to provide the implant. During the step of additive manufacturing, the method may comprises selective laser sintering, in which at least one portion of the article is sintered. In some embodiments, the method comprises a step of cleaning the article to remove excessive particles before an optional step of sintering the article at the elevated temperature.

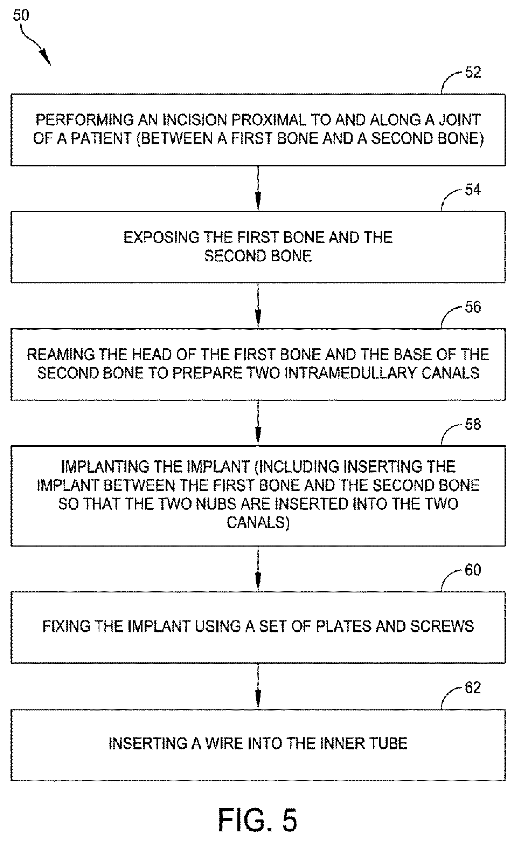

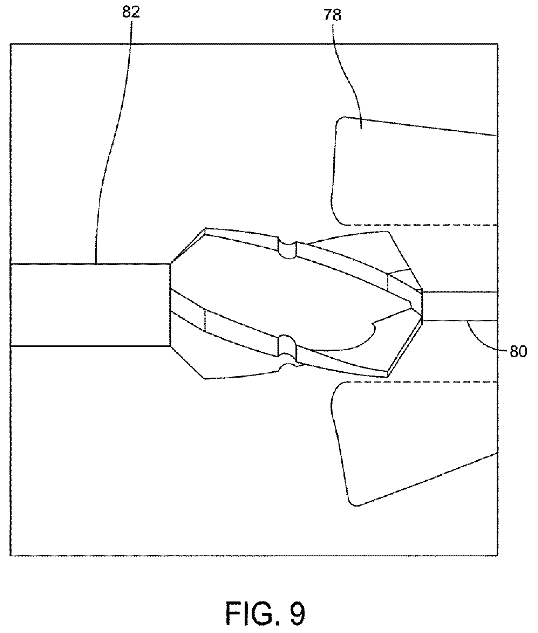

In another aspect, the present disclosure also provides a method of using an implant, for example, the implant as described above, to fuse, fix or partially replace a joint of a patient between a first bone having a head and a second bone having a base. For example, an exemplary method is described in FIGS. 5-13. The method comprising the following steps: performing an incision proximal to and along the joint of the patient, exposing the head of the first bone and the base of the second bone, reaming the head of the first bone and the base of the second bone to prepare two intramedullary canals including a first canal in the first bone and the second canal in the second bone.

The method further comprises a step of implanting an implant between the head of the first bone and the base of the second bone. The implant comprises a body having a cylindrical portion extending from a first end to a second end, a first nub extending from the first end of the cylindrical portion, and a second nub extending from the second end of the cylindrical portion. In the step of implanting the implant, the implant is inserted between the first bone and the second bone so that the two nubs of the implant are inserted into the two intramedullary canals, and two surfaces of the cylindrical portion on the first and the second ends are disposed on cortical rims of the first bone and the second bone.

In some embodiments, the method further comprises fixing the implant using a set of plates and screws during or after the step of implanting the implant. In an exemplary surgical procedure, a wire (for example, k-wire) may be inserted into the inner layer during the step of implanting the implant. In some embodiments, the implant is configured to fuse a metatarsophalangeal joint. The first bone may be a metatarsal bone. The second bone may be a proximal phalange in the same toe of the patient.

The implant provided in the present disclosure provides optimal shape and size, and excellent alignment with bones, and also provides excellent biocompatibility. For example, the implant allows bone growth from one end of the implant and the other end without interference of any soft tissue. The porous structure and/or rough surface described above promote bone ingrowth and fixation while the solid structure and smooth surface described above prevent tissue ingrowth.

### BRIEF DESCRIPTION OF THE DRAWINGS

The present disclosure is best understood from the following detailed description when read in conjunction with the accompanying drawings. It is emphasized that, according to common practice, the various features of the drawings are not necessarily to scale. On the contrary, the dimensions of the various features are arbitrarily expanded or reduced for clarity. Like reference numerals denote like features throughout specification and drawings.
FIG. 1 is a perspective view of an exemplary implant in accordance with some embodiments.
FIG. 2 is a sectional view or a projectional view from one end of the exemplary implant of FIG. 1.
FIG. 3 is a side (or plan) view of the exemplary implant of FIG. 1.
FIG. 4 is a flow chart diagram illustrating an exemplary method of making an implant in accordance with some embodiments.
FIG. 5 is a flow chart diagram illustrating an exemplary surgical procedure as a part of a method of using an implant in accordance with some embodiments.
FIGS. 6-13 illustrate various steps of an exemplary surgical procedure of FIG. 5 in accordance with some embodiments.

### DETAILED DESCRIPTION

This description of the exemplary embodiments is intended to be read in connection with the accompanying drawings, which are to be considered part of the entire written description. In the description, relative terms such as "lower," "upper," "horizontal," "vertical,", "above," "below," "up," "down," "top" and "bottom" as well as derivative thereof (e.g., "horizontally," "downwardly," "upwardly," etc.) should be construed to refer to the orientation as then described or as shown in the drawing under discussion. These relative terms are for convenience of description and do not require that the apparatus be constructed or operated in a particular orientation. Terms concerning attachments, coupling and the like, such as "connected," refer to a relationship wherein structures are secured or attached to one another either directly or indirectly through intervening structures, as well as both movable or rigid attachments or relationships, unless expressly described otherwise.

In the present disclosure the singular forms "a," "an," and "the" include the plural reference, and reference to a particular numerical value includes at least that particular value, unless the context clearly indicates otherwise. When values are expressed as approximations, by use of the antecedent "about," it will be understood that the particular value forms another embodiment. As used herein, "about X" (where X is a numerical value) preferably refers to ±10% of the recited value, inclusive. For example, the phrase "about 8" preferably refers to a value of 7.2 to 8.8, inclusive. Where present, all ranges are inclusive and combinable. For example, when a range of "1 to 5" is recited, the recited range should be construed as including ranges "1 to 4", "1 to 3", "1-2", "1-2 & 4-5", "1-3 & 5", "2-5", and the like. In addition, when a list of alternatives is positively provided, such listing can be interpreted to mean that any of the alternatives may be excluded, e.g., by a negative limitation in the claims. For example, when a range of "1 to 5" is recited, the recited range may be construed as including situations whereby any of 1, 2, 3, 4, or 5 are negatively excluded; thus, a recitation of "1 to 5" may be construed as "1 and 3-5, but not 2", or simply "wherein 2 is not included." It is intended that any component, element, attribute, or step that is positively recited herein may be explicitly excluded in the claims, whether such components, elements, attributes, or steps are listed as alternatives or whether they are recited in isolation.

For brevity, "orthopedic implant devices," "implant" and the like are used interchangeably in the present disclosure. References to "prosthetic implant devices," or "implant" made in the present disclosure will be understood to encompass any suitable device configured to fuse, fix or partially replace a joint between two bones, including but not limited to a MTP implant.

References to "solid" or "substantially solid" are made relative to references to "porous" and "substantially porous." Unless expressly indicated otherwise, references to "solid" or "substantially solid" made below will be understood to describe a material or structure having 0-5% by volume (e.g., 0-2% by volume) of porosity. A small amount of pores, particularly closed pores, may be embedded inside a solid or substantially solid material.

Unless expressly indicated otherwise, references to "porous" or " substantially porous" made below will be understood to describe a material or structure having a significant amount of pores, for example, higher than 5% by volume of porosity. A porous or substantially porous materials may have pores, particularly open pores on the surface. The porosity on or adjacent to the surface may be higher than 5% by volume in some embodiments. When a material monolith is porous, the porosity may be in the range from 20-95% (e.g., 50-80%) by volume.

All the data of pore size and porosity were measured following the FDA's guidance: "Guidance Document for Testing Orthopedic Implants With Modified Metallic Surfaces Apposing Bone or Bone Cements," 1994. Each part was sectioned using electric discharge machining to produce smooth and even surfaces that represent cross-sections through the porous material. Green modeling clay was used to fill the pores of the cut face. A razor blade was used to remove any excess modeling clay from the cross section. Images were taken at 75x magnification using a Zeiss microscope with a camera attachment. Parts were oriented in a way to give best possible color contrast between the titanium and the modeling clay. Simagis image analysis software (Smart Imaging Technology, Houston, TX) was used to determine the percent porosity, strut diameter, interconnecting pore diameter and pore cell diameter. The pore size (or interconnecting pore size) was defined as the approximately circular pore opening that connects larger pore cells.

Referring to FIGS. 1-3, an exemplary implant 10 comprises a body 11 (i.e. combinations of 12, 14, and 16) having a cylindrical portion 12 extending from a first end 12-1 to a second end 12-2, a first nub 14 extending from the first end 12-1 of cylindrical portion 12, and a second nub 16 extending from the second end 12-2 of cylindrical portion 12. Body 11 in of implant 10 may be a unitary body in some embodiments. References to "nub" are understood to describe a small part sticking out from one end of implant 10. A nub can be also referred as a "knob," or "nugget."

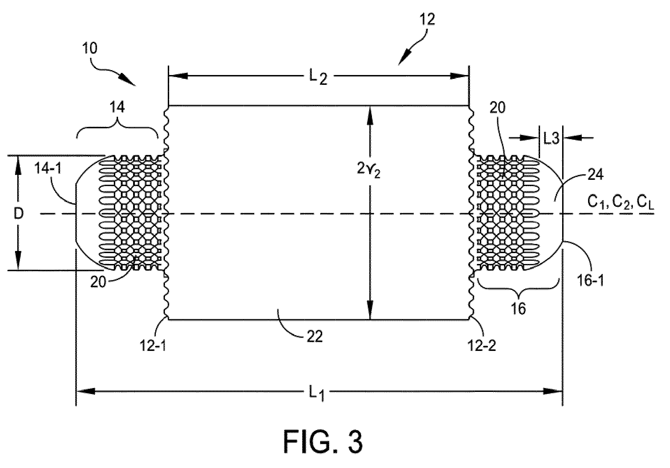

Based on the material composition or physical structure, body 11 includes an inner layer 18, an outer layer 22, and a middle layer 20. Inner layer 18, outer layer 22, and middle layer 20 may be made of the same material having the same chemical composition but different physical structures in some embodiments. Inner layer 18 has a first length (L1) extending from a tip 14-1 of the first nub 14 to a tip 16-1 of the second nub 16, and has a first central axis (C1) that is collinear with a central longitudinal axis (CL) of implant 10. Outer layer 22 provides an outer surface 22-1 of implant 10. As shown in FIG. 2, inner layer 18 has an outer radius (a first radius) r1, while the outer layer 22 has an outer radius (a second radius) r2. The first radius r1 is smaller than the second radius r2 in some embodiments. As shown in FIG. 3, outer layer 22 has a second length (L2) extending from the first end 12-1 to the second end 12-2 of cylindrical portion 12, and has a second central axis (C2) that is collinear with the central longitudinal axis (CL) of implant 10. As shown in FIG. 3, the first length (L1) for inner layer 18 is longer than the second length (L2) for outer layer 22 in some embodiments. Inner layer 18 is cylindrical and hollow as it defines a hole 19 in the middle along the first central axis (C1) in some embodiments.

Middle layer 20 is disposed between inner layer 18 and outer layer 22. Middle layer 20 comprises a porous material in some embodiments. The inner layer 18 and at least part of middle layer 20 collectively provide the two nubs 14, 16. As shown in FIGS. 1 and 3, middle layer 20 has portions protruding from the first end 12-1 and the second end 12-2 of cylindrical portion 10 to provide nubs 14, 16.

In some embodiments, inner layer 18 and outer layer 22 have a solid or substantially solid structure. For example, inner layer 18 and outer layer 22 can be made of a material having no porosity or a porosity of less than 5% by volume (e.g., 0-2%). Outer layer 22 has an outer circumferential surface 22-1 that is substantially smooth, for example, having a roughness parameter smaller than 5 microns. Inner layer 18 has an inner circumferential surface 18-1 and two end surfaces 18-2 substantially smooth, for example, having a roughness parameter smaller than 5 microns. Each of inner layer 18 and outer layer 22 comprises a metal such as, for example, titanium, titanium alloy, or stainless steel to list only a few possible metals. In some embodiments, such a metal is titanium, or titanium alloy. The titanium can be of high purity, for example, 95-100%. In some embodiments, each of inner layer 18 and outer layer 22 is made of pure and solid titanium, or solid titanium alloy. In some embodiments, outer layer 22 has two end surfaces being rough. The end surfaces of outer layer 22 may be knurled, matted or patterned in some embodiments. The end surfaces of outer layer 22 may have some small protuberances.

In some embodiments, middle layer 20 comprises two portions. As best seen in FIG. 2, the first portion 20-1 is a portion ("middle portion" or "sandwiched portion") 20-1, which extends from the first end 12-1 to the second end 12-2 of cylindrical portion 12, and is sandwiched between inner layer 18 and outer layer 22. The second portion is the at least one portion 20-2 of middle layer 20 providing the two nubs 14, 16. In some embodiments, the at least one portion 20-2 of middle layer 20 providing nubs 14, 16 is porous. The outer surfaces of the at least one portion 20-2 of middle layer 20 may be rough, knurled, matted or patterned in some embodiments. The outer surfaces of the at least one portion 20-2 of middle layer 20 may have some small protuberances. The middle portion or sandwiched portion 20-1 can be porous or solid. In some embodiments, the middle layer 20 is one unitary layer comprising a porous material. The middle layer 20 may comprise porous titanium or titanium alloy. The porous material of the middle layer 20 may have pores of any suitable size or ranges. For example, The pore size may be in the range of from about 1 micron to about 2000 microns in diameter, for example, from about 50 microns to about 1000 microns in diameter, or in the range of from about 400 microns to about 600 microns in diameter. The pores can be continuous and open. The porosity can be in the range from about 20 % to about 90 % (e.g., from about 50 % to about 80%) by volume in some embodiments.

In some embodiments, the whole middle layer 20 is made of porous titanium such as, for example, BIOFOAM® material available from Wright Medical Inc, although other porous materials can be used. BIOFOAM® material is made of titanium and has pores, for example, of roughly 500 microns in diameter. The porosity can be up to 70% by volume. Such porous titanium has continuous and open pores. Such porous titanium may have a compression strength, for example, in the range of from about 50 to about 100 MPa.

Middle layer 20 may have a closed nonporous structure to promote bone fixation through friction and bone ingrowth. In some embodiments, middle layer 20 has at least one exposed surface having a predetermined surface roughness, which might be in any suitable range. The surface roughness parameter may be equal to or higher than 5 micron, or higher than 10 or 20 microns. In some embodiments, the middle layer may have a region 24 with a smooth surface adjacent to the tip 14-1, 16-1 of nubs 14, 16, which is also adjacent to the end surfaces 18-2 of the inner layer 18 as best seen in FIG. 1.

Implant 10 described above can be used as a metatarsophalangeal (MTP) implant configured to fuse a metatarsophalangeal joint of a patient. However, one of ordinary skill in the art will understand that implant 10 can be used to fuse, fix or partially replace another joint between two adjacent bones.

The implant can be of any suitable size, which can be determined by the size of the joint and associated bones. Table 1 lists some examples of implants for MTP joint fusion.

**Table 1. Exemplary MTP Implants of Different Sizes**

| Example | Diameter of Cylindrical portion (2*r₂) (mm) | Length of Cylindrical Portion (L2) (mm) | Length of a nub (L1-L2) (mm) | Diameter of nub (D) (mm) |
|---|---|---|---|---|
| 1 | 15 | 5 | 7.5 | 8 |
| 2 | 19 | 5 | 7.5 | 8 |
| 3 | 15 | 10 | 7.5 | 8 |
| 4 | 19 | 10 | 7.5 | 8 |
| 5 | 15 | 17 | 7.5 | 8 |
| 6 | 19 | 17 | 7.5 | 8 |
| 7 | 15 | 24 | 7.5 | 8 |
| 8 | 19 | 24 | 7.5 | 8 |

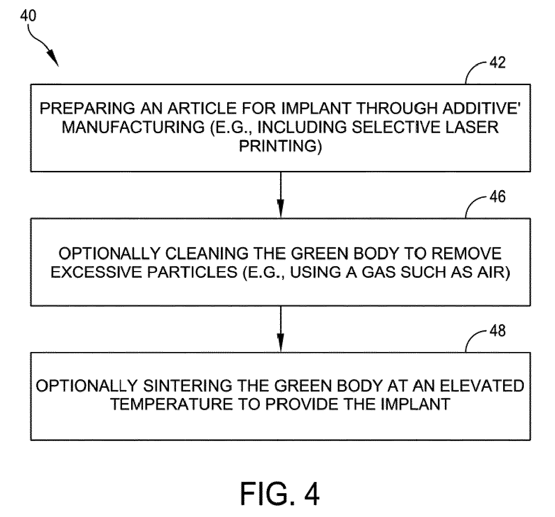

Referring now to FIG. 4, one example of a method 40 for making exemplary implant 10 is described.

At step 42, an article for an implant 10 is prepared. In some embodiments, the article for implant is prepared using a suitable method, for example, using an additive manufacturing technique. The article can be also made through three-dimensionally (3-D) printing. The article is similar to or about the same as the final implant 10, with consideration of possible shrinkage in the later sintering processes. Computer-aided design (CAD) / Computer-aided manufacturing (CAM) technologies can be used in combination with the additive manufacturing technique. An exemplary implant 10 can be designed using CAD. A model including related design parameters can be output from a computer. The related design parameters for implant 10 as a final product include shape, configuration, dimensions, porosity, and surface roughness of each portion of implant 10.

Any equipment suitable for additive manufacturing of metals can be used at step 42. Physical parameters of the article implant such as porosity and density of the material in each location can be correspondingly adjusted by the additive manufacturing equipment. Examples of the material used include but are not limited a metal powder such as titanium, titanium alloy, cobalt chromium alloy or stainless steel. Examples of a suitable additive manufacturing equipment is available from, for example, EOS of Germany and Arcam of Sweden to list only two possible examples. Selective laser sintering is applied while or right after each point or portion is printed. Direct laser sintering or selective later sintering may be used. One of ordinary skill in the art will understand that other sintering methods can be used.

At step 46, the article is cleaned to remove excessive particles, which are not attached with or are loosely attached to the article. Step 46 may be optional, and may be performed by applying high pressure air or other gases to the surface of the article. The excessive particles can be blown away.

At step 48, which is optional, the article is sintered at an elevated temperature to provide the implant 10 described above. Such a sintering can be performed in an oven or furnace. The heat sintering can be performed at any suitable temperature. The heat sintering of titanium may be performed at a temperature, for example, in the range from about 1000 to about 1500 C°. The temperature and time can be selected to control the physical parameters of final implant 10. Resulting implant 10 provides excellent strength and stiffness.

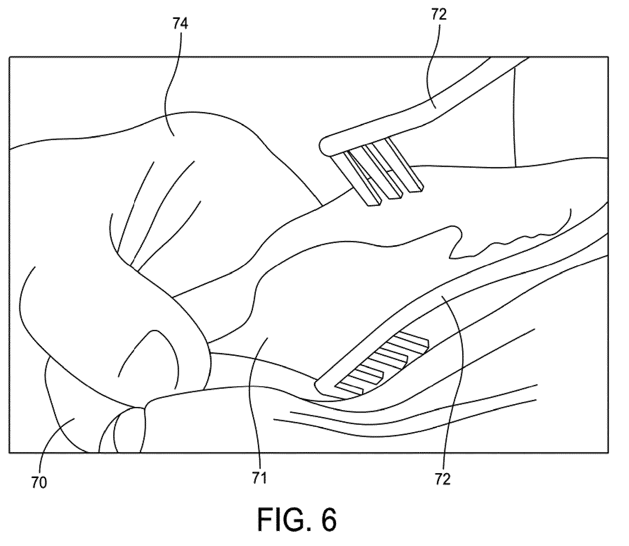

Referring now to FIG. 5, one example of a method 50 of implant is now described. As described with reference to FIGS. 5-13, the implant 10 is used to fuse a joint of a patient between a first bone 68 having a head 78 and a second bone 69 having a base 79, such as an MTP joint, although one of ordinary skill in the art will understand that implant 10 can be used to replace other joints. Thus, in the exemplary method 50 illustrated in FIGS. 6-13, the first bone 68 is a metatarsal bone, and the second bone 69 is a proximal phalange in a same toe of the patient.

At step 52, an incision is performed proximal to and along the joint of the patient. FIG. 6 illustrates step 52 for a MTP joint fusion.

When a MTP implant is used in a MTP joint, a dorsal longitudinal or dorsal medial incision can be used as a surgical approach. The toe 70 to be operated upon can be grabbed by a hand 74 of a medical professional during the surgery. Tools such as retractors 72 also may be used. The incision can be made along a metatarsal bone. The incision can be made proximal to the interphalangeal joint, and extended over the dorsum of the MTP joint medial to the extensor hallucis longus (EHL) tendon. The incision may end on the medial aspect of the metatarsal, 2-3 cm proximal to the joint.

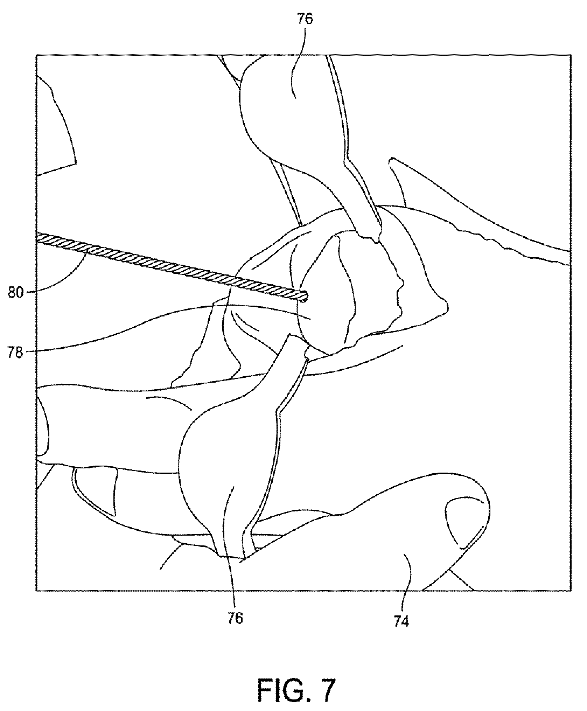

At step 54, the head 78 of the first bone 68 and the base 79 of the second bone 69 are exposed. FIG. 7 illustrates an exemplary MTP joint at step 54.

Collateral ligaments in the joint capsule 71 can be incised and released to expose the base of the proximal phalanx and the metatarsal head. The phalanx plantarly can be also displaced before exposing the metatarsal head 78. Suitable tools such as retractors 76 can be used to expose the head 78. With a powered drill, a K-wire (Kirschner wire) 80 is placed proximally through the center of the metatarsal head 78. With a Jacobs chuck, a Cannulated AO Quick Connect can be attached to the power driver and connect a female reamer 82. Reamer 82 is placed over K-Wire 80 and gently ream the metatarsal head until bleeding subchondral bone becomes visible on the joint surface. K-wires used in the present disclosure may not be the same K-wire in each step. Reamer 82 is used to prepare the intramedullary canal for the nub of the implant.

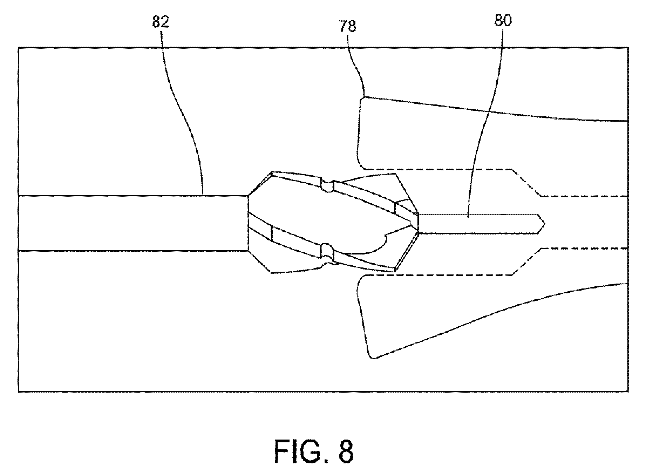

At step 56, the head 78 of the first bone 68 and the base 79 of the second bone 69 are reamed to prepare two intramedullary canals, including a first canal in the first bone 68 and the second canal in the second bone 69. FIGS. 8-9 illustrate step 56 during a MTP joint fusion. Bones 84 are from other toes of the same foot where the toe 70 of operation is located. Reaming of the phalanx (second bone 69) is performed in a similar fashion to the metatarsal head. To properly expose the articular surface of the phalanx 69, the phalanx 69 is elevated dorsally and distally away from the metatarsal head 78. A curved retractor can be used. A K-Wire 80 is again placed in the center of the articular cartilage and directed through diaphysis. Care can be taken not to remove too much bone or damage the metatarsal head 78. In some embodiments, reamers for both the metatarsal and phalangeal side may have the same diameter. Either of male and female spherical reamers may be used for preparation of the MTP joint surfaces.

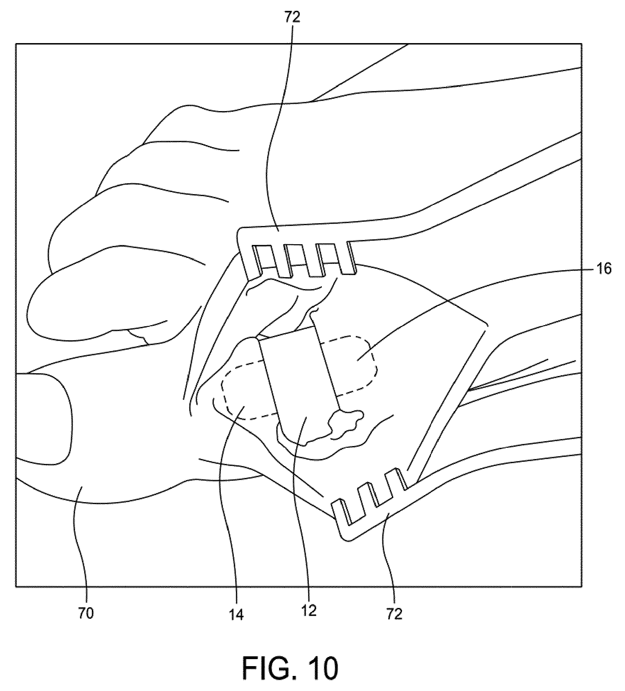

At step 58, implant 10 is implanted between the head of the first bone 68 and the base of the second bone 69. FIG. 10 illustrates the foot implanted with exemplary implant 10. As described in FIGS. 1-3, implant 10 comprises body 11 having a cylindrical portion 12 extending from a first end 12-1 to a second end 12-2, a first nub 14 extending from the first end 12-1 of cylindrical portion 12, and a second nub 16 extending from the second end 12-2 of cylindrical portion 12. As shown in FIG. 10, implant 10 is inserted between the first bone 68 and the second bone 69 so that the two nubs 14, 16 of implant 10 are inserted into the two intramedullary canals, and two end surfaces of cylindrical portion 12 on the first end 12-1 and the second end 12-2 are disposed on cortical rims of the first bone 68 and the second bone 69.

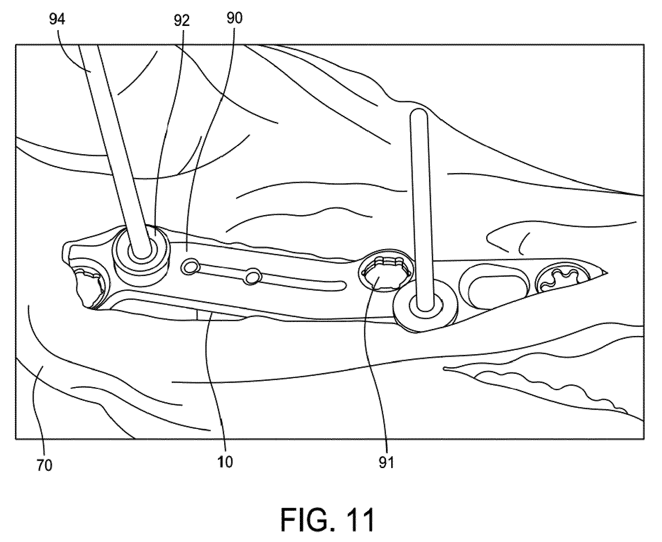

At step 60, implant 10 is fixed using a set of fusion plates 90 and screws 92 in some embodiments. FIG. 11 illustrates the structure after step 60. Step 60 is performed during or after step 58. Ancillary fixation 94 can be also used to temporarily hold the plate 90 in place through the holes 91 in the plate 90. A drill can be used to drill holes through the cortices of the bones. Fusion plate 90 can be optionally secured with one or more screws 92 in some embodiments.

At step 62, a wire (for example, k-wire) is inserted into the inner layer 18 or other locations. Step 62 may be optional, and may be performed during step 58 of implanting the implant 10. All K-wires are removed when plate 90 is securely fixed. Surgical closure is then performed in the normal fashion.

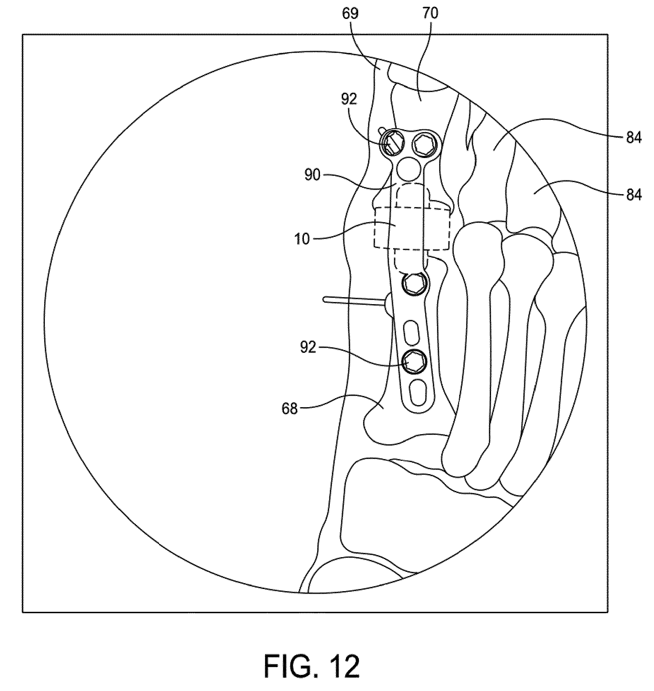

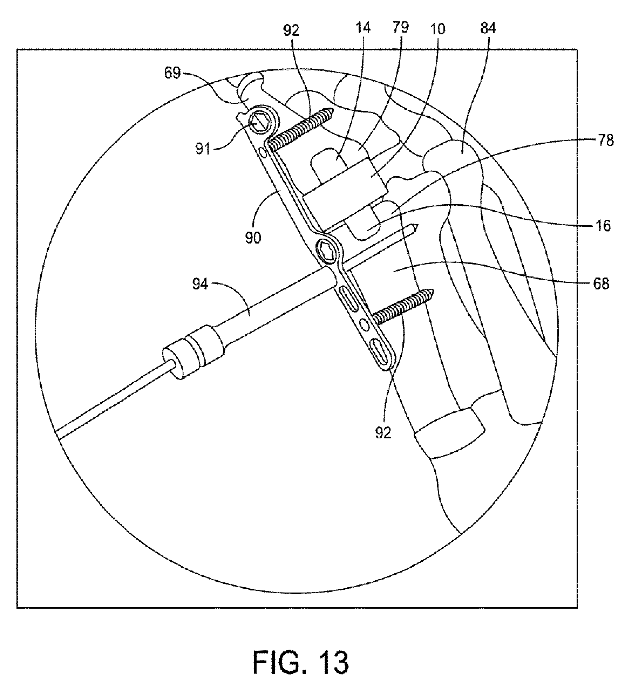

FIG. 12-13 illustrate fluoroscopic images of implant 10 with plate 90 implanted in a human foot. FIG. 12 is a top-down view while FIG. 13 is a side view.

The implant described herein advantageously is the optimal shape and size for the MTP joint such that the implant provides better alignment with bones, and also provides better biocompatibility. For example, the implant allows bone growth from one end of the implant to the other end without interference of any soft tissue. The porous structure and/or rough surface described above promote bone ingrowth and fixation while the solid structure and smooth surface described above prevent tissue ingrowth.

In accordance with some embodiments, during an implanting surgery of an MTP implant, nubs 14, 16 of implant 10 are inserted into intramedullary canals in the metatarsal bone 68 and the base of the proximal phalange 69, and the surfaces of both ends of the middle body rests on the cortical rim of each bone. Solid inner layer 18 allows for easy insertion of a k-wire during the surgery and also enhances strength of the MTP implant. The nubs 14, 16 and both ends of body 11, which are made of porous titanium (e.g., BIOFOAM®), have a roughed surface to promote bone fixation through friction and bone ingrowth. This configuration allows bone growth from one end of the MTP implant to the other end without interference of any soft tissue. Solid outer layer of the body also prevents possible growth of soft tissue thereon. A set of plates and screws are used for fusion of the MTP joint in some embodiments. Fusion of this joint is most often performed for treatment of end-stage hallux rigidus, severe Hallux Valgus, rheumatoid and post-traumatic arthritis, and for revision of nonunions. The combination of plates and screws provides for a very stiff and stable construct, and ensures that the hallux is fused in proper anatomic alignment.

Exemplary method 50 provides rapid fusion of the joint without excessive shortening of the toe or removal of structural bone, and correct orientation of the MTP joint for natural gait biomechanics and footwear comfort. Exemplary method 50 also enables a high fusion rate and early return to function by creating a very stable fusion construct.

Although the subject matter has been described in terms of exemplary embodiments, it is not limited thereto. Rather, the appended claims should be construed broadly, to include other variants and embodiments, which may be made by those skilled in the art.

## Claims

1. An implant, comprising a body having a cylindrical portion extending from a first end to a second end, a first nub extending from the first end of the cylindrical portion, and a second nub extending from the second end of the cylindrical portion,
wherein the body includes:
an inner layer having a first length extending from a tip of the first nub to a tip of the second nub and having a first central axis that is collinear with a central longitudinal axis of the implant,
an outer layer having a second length extending from the first end to the second end and having a second central axis that is collinear with the central longitudinal axis of the implant, the outer layer providing an outer surface of the implant, and
a middle layer comprising a porous material disposed between the inner layer and the outer layer, and
wherein the inner layer and at least one portion of the middle layer provide the two nubs.

2. The implant of claim 1, wherein the inner layer and the outer layer have a solid structure.

3. The implant of claim 1 or claim 2, wherein the outer layer has an outer circumferential surface, and the inner layer has an inner circumferential surface and two end surfaces.

4. The implant of any one of the preceding claims, wherein each of the inner layer and the outer layer comprises a metal.

5. The implant of claim 4, wherein the metal includes titanium.

6. The implant of any one of the preceding claims, wherein the at least one portion of the middle layer providing the two nubs is porous, and a portion of the middle layer extending from the first end to the second end of the cylindrical portion of the body and sandwiched between the inner layer and the outer layer is porous or solid.

7. The implant of any one of the preceding claims, wherein the middle layer comprises porous titanium.

8. The implant of any one of the preceding claims, wherein the porous material in the middle layer has pores in the range of from about 50 microns to about 1000 microns in diameter.

9. The implant of any one of the preceding claims, wherein the porous material in the middle layer comprises titanium having pores in the range of from about 400 microns to about 600 microns in diameter.

10. The implant of any one of the preceding claims, wherein the middle layer has at least one exposed surface having a predetermined surface roughness and configured to promote bone fixation through friction and bone ingrowth.

11. The implant of any one of the preceding claims, wherein the middle layer has a region with a smooth surface adjacent to the inner layer.

12. The implant of any one of the preceding claims, wherein the implant is a metatarsophalangeal (MTP) implant configured to replace a metatarsophalangeal joint of a patient.

13. A method for making an implant according to any one of the preceding claims, the method comprising forming an article for the implant through an additive manufacturing technique.

14. The method of claim 13, wherein the step of forming the article for the implant comprises selective laser sintering at least one portion of the article.

15. The method of claim 13 or claim 14, further comprising sintering the article at an elevated temperature to provide the implant.
